# EUROPEAN PATENT SPECIFICATION

(11) **EP 1 010 755 B1**
(45) Date of publication and mention of the grant of the patent: **06.10.2010**
(21) Application number: 99125302.2
(22) Date of filing: 17.12.1999
(51) Int. Cl.: C12N 1/21, C12P 13/14, C12N 15/11, C12N 15/53

(54) **Method for producing L-Glutamic acid by fermentation**
Verfahren zur fermentativen Herstellung von L-Glutaminsäure
Procédé de production d'acide L-glutamique par fermentation

(30) Priority: 18.12.1998 JP 36061998
(43) Date of publication of application: 21.06.2000
(73) Proprietor: Ajinomoto Co., Inc., Tokyo 104-8315 (JP)
(72) Inventor: Kanno, Sohei, c/o Ajinmoto Co., Inc., Kawasaki-ku, Kawasaki-shi, Kanagawa (JP); Kimura, Eiichiro, c/o Ajinmoto Co., Inc., Kawasaki-ku, Kawasaki-shi, Kanagawa (JP); Matsui, Kazuhiko, c/o Ajinmoto Co., Inc., Kawasaki-ku, Kawasaki-shi, Kanagawa (JP); Kurahashi, Osamu, c/o Ajinmoto Co., Inc., Kawasaki-ku, Kawasaki-shi, Kanagawa (JP); Horino, Issei, c/o Ajinmoto Co., Inc., Kawasaki-ku, Kawasaki-shi, Kanagawa (JP); Nakamatsu, Tsuyoshi, c/o Ajinmoto Co., Inc., Kawasaki-ku, Kawasaki-shi, Kanagawa (JP)
(74) Representative: HOFFMANN EITLE

(56) References cited:
- US-A- 5 846 790
- CHEMICAL ABSTRACTS, vol. 122, no. 17, 24 April 1995 (1995-04-24) Columbus, Ohio, US; abstract no. 209426, ROLLIN, CATHERINE ET AL: "13C-NMR studies of Corynebacterium melassecola metabolic pathways" XP002134304 & EUR. J. BIOCHEM. (1995), 227(1/2), 488-93 ,
- DATABASE WPI Section Ch, Week 198841 Derwent Publications Ltd., London, GB; Class B05, AN 1988-290579 XP002134305 & JP 63 214189 A (ASAHI CHEM IND CO LTD), 6 September 1988 (1988-09-06)

### SEQUENCE LISTING

<110> Ajinomoto Co., Inc.
<120> Method for producing L-glutamic acid by Fermentation
<130> OP941
<141> 1999-12-
<150> JP 10-360619
   <151> 1998-12-18
<160> 16
<170> PatentIn Ver. 2.0
<210> 1
   <211> 33
   <212> DNA
   <213> Artificial Sequence
<220>
   <223> Description of Artificial Sequence:primer for amplifying Esherichia coli pyruvate dehydrogenase gene
<400> 1
   ggctctagag cgagagttca atgggacagg ttc 33
<210> 2
   <211> 33
   <212> DNA
   <213> Artificial Sequence
<220>
   <223> Description of Artificial Sequence:primer for amplifying Esherichia coli pyruvate dehydrogenase gene
<400> 2
   ggcctcgagg gaaaaagtac agcagcgccc act 33
<210> 3
   <211> 23
   <212> DNA
   <213> Artificial Sequence
<220>
   <223> Description of Artificial Sequence:primer for amplifying Brevibacterium lactofermentum pdhA
<220>
   <221> misc_feature
   <222> (3,6,8,15,18,20)
   <223> n=inosine
<400> 3
   acngtntcna tgggnctngg ncc 23
<210> 4
   <211> 23
   <212> DNA
   <213> Artificial Sequence
<220>
   <223> Description of Artificial Sequence: primer for amplifying Brevibacterium lactofermentum pdhA
<220>
   <221> misc_feature
   <222> (6,12,15,18,20)
   <223> n=inosine
<400> 4
   ccttcnccgt tnagngtngt ncg 23
<210> 5

   <211> 30
   <212> DNA
   <213> Artificial Sequence
<220>
   <223> Description of Artificial Sequence: primer for LA in vitro cloning of Brevibacterium lactofermentum pdhA gene
<400> 5
   ttgcagttaa ccacgaaggt caggttgtcc 30
<210> 6
   <211> 30
   <212> DNA
   <213> Artificial Sequence
<220>
   <223> Description of Artificial Sequence: primer for LA in vitro cloning of Brevibacterium lactofermentum pdhA gene
<400> 6
   tggatgagac cacgtgattc tggctcgtcc 30
<210> 7
   <211> 30
   <212> DNA
   <213> Artificial Sequence
<220>
   <223> Description of Artificial Sequence: primer for LA in vitro cloning of Brevibacterium lactofermentum pdhA gene
<400> 7
   acagatcctg cacgaaggca tcaacgaggc 30
<210> 8
   <211> 30
   <212> DNA
   <213> Artificial Sequence
<220>
   <223> Description of Artificial Sequence: primer for LA in vitro cloning of Brevibacterium lactofermentum pdhA gene
<400> 8
   tcatcgctgc gggtacctcc tacgccaccc 30
<210> 9
   <211> 5370
   <212> DNA
   <213> Brevibacterium lactofermentum
<220>
   <221> CDS
   <222> (2360)..(5125)
<400> 9
   tcacgttacg gcgatcaaca ccgcaaccac tacgagaaga tctccaaacg agaccaagag 60
   cgcttctaag cccgtctcat tttgcacctg ccattctgtg aggatatggc aggtgctttt 120
   tcatgccact atcttggggt tctcggtatt agatcttctg ataaaaaccc gatagttttc 180
   ttgcgctaga cactaattac ggcaccgctt aagcatggtc gtgacacgta aaacctgact 240
   taggccattt tgatgtggtg tagatcatat tgacgtcaat gaatgaagtg actaactccg 300
   ccgaatccac atcgtctaaa aggcctgggc gaccacgtaa agacgggcac gacgagaaga 360
   tcatcgacgc aactttacgg ctcatcgaca gcaatcgtcc cgtcacggtc aatgcagttg 420
   tcaaagaaag cggagtggca cgtgcagcgg tttatcgacg ctggcccagg ctagtggatc 480
   tagtagcgga agctttagat gccgggcgag ctccagttga aatagatacc ccaggggaca 540
   tcaaagagac cttgattgat gggctgttta caaatcaggc gaaaaccact ggagtctcct 600
   atcctcgtca gcgatttcgc aaacggctcg agttggtgat gtcagatcaa gaattacagc 660
   tcgcctaatg gaattcacat gtgaagagac gtcgagaagc aaatattcgc gcgctgcaag 720
   tcgcgcaaga aaaaggccaa atccgggcgg atctagacat cgaggcgtgc ctcgatgcaa 780
   tccttggggt gttttattac caatcggtcg cgcgtggagt aaatttcacc gaccaaggta 840
   caacgcaacg atgcagagaa gccttggagg tgatctggca tggaatggaa ccttaaattc 900
   aggttctgac gaggtgcgaa gcaagttgtc gcgcgccgca cctcagtatc cggatcaact 960
   taatttcgaa gtgctgggtt ttctcgcgca tacccaatgc gtaccgatgt gcccatgagc 1020
   gaaaaacagg ccacgataag tttcttaaaa cttatcgtgg cctgcttcta tatttgtgcg 1080
   ccctgacggg ctcgaaccgc cgacctgctg ggtgtaaacc agctgctctt ccagctgagc 1140
   taaaggcgcg cacgtgcttt tctagaacca ccttggtggc ctcgaaagca acgagtgaaa 1200
   tactaacaca caatctccac agacctaaaa tcgctgctca ggccgtggaa attagcgatt 1260
   gttaaggctt cttgtttcca cgctggacga ggcaagaacc ttgccaatta ccgagacgtt 1320
   ccgccttggt ctgcacgaga cctgccagtt gtgctgattc agagataact ccaggagcca 1380
   gggctccttc tttaccaatg ccaggagtca acacccagat acgaccattc tcagcgaggg 1440
   agcggatgga atccacaagt ccgtcgacga gatcgccgtc atcctcgcgc caccagagca 1500
   gcacgacatc gcacagctcg tcggtttctt catcgagtag ttcctcaccg attgcatctt 1560
   cgatggactc gctgatcagc gtgtcggaat cttcatccca tccaatttct tgaacgatat 1620
   gacccgattg aatgccgagt agttgagcat aatcctgggc accttgcttg actgcgcccg 1680
   gagcgtcggc cactttaata atcctcctcg tgtgggcccc gatgtgtttt tcgattacat 1740
   ggattcaaca tgaaaccgcg gggctattga tatatccgaa ttgcacatta ccgtccaacc 1800
   ggtactttga accacctttc cctggaattt tttccttttc ctcccccttt acgctcaaga 1860
   atcaatgaat tcaatcactg gccagcgatt aacttttcga gttttcagtc ttggatttcc 1920
   acaattctct tcaaaataat ggtggctaga tttttcatca aaccctcacc aaaaggacat 1980
   cagacctgta gttttatgcg attcgcgtca aacgtgagag aaacatcaca tctcacggga 2040
   aactacccga taattctttg caaaactttg caaagggtaa tgaacatgca gctagtttcc 2100
   gtagaaatgt tctttaaaaa atccacaaca attgccagga agcacaccga ttgatggata 2160
   cctgaaatcc cagtgagcgc accactcccc ttacgtcaca gtctgtaaaa caaatcttcg 2220
   gtgttgcgta tccttgttaa taacttatgc gttgacccat tcgtgcactt cggtgtgcca 2280
   caattaggta cgaccaagaa tgggaccggg aaaccgggac gtataaacga aataaaacat 2340
   tccaacagga ggtgtggaa atg gcc gat caa gca aaa ctt ggt ggt aag ccc 2392
   Met Ala Asp Gln Ala Lys Leu Gly Gly Lys Pro
   1 5 10
   tcg gat gac tct aac ttc gcg atg atc cgc gat ggc gtg gca tct tat 2440
   Ser Asp Asp Ser Asn Phe Ala Met Ile Arg Asp Gly Val Ala Ser Tyr
   15 20 25
   ttg aac gac tca gat ccg gag gag acc aac gag tgg atg gat tca ctc 2488
   Leu Asn Asp Ser Asp Pro Glu Glu Thr Asn Glu Trp Met Asp Ser Leu
   30 35 40
   gac gga tta ctc cag gag tct tct cca gaa cgt gct cgt tac ctc atg 2536
   Asp Gly Leu Leu Gln Glu Ser Ser Pro Glu Arg Ala Arg Tyr Leu Met
   45 50 55
   ctt cgt ttg ctt gag cgt gca tct gca aag cgc gta tct ctt ccc cca 2584
   Leu Arg Leu Leu Glu Arg Ala Ser Ala Lys Arg Val Ser Leu Pro Pro
   60 65 70 75
   atg acg tca acc gac tac gtc aac acc att cca acc tct atg gaa cct 2632
   Met Thr Ser Thr Asp Tyr Val Asn Thr Ile Pro Thr Ser Met Glu Pro
   80 85 90
   gaa ttc cca ggc gat gag gaa atg gag aag cgt tac cgt cgt tgg att 2680
   Glu Phe Pro Gly Asp Glu Glu Met Glu Lys Arg Tyr Arg Arg Trp Ile
   95 100 105
   cgc tgg aac gca gcc atc atg gtt cac cgc gct cag cga cca ggc atc 2728
   Arg Trp Asn Ala Ala Ile Met Val His Arg Ala Gln Arg Pro Gly Ile
   110 115 120
   ggc gtc ggc gga cac att tcc act tac gca ggc gca gcc cct ctg tac 2776
   Gly Val Gly Gly His Ile Ser Thr Tyr Ala Gly Ala Ala Pro Leu Tyr
   125 130 135
   gaa gtt ggc ttc aac cac ttc ttc cgc ggc aag gat cac cca ggc ggc 2824
   Glu Val Gly Phe Asn His Phe Phe Arg Gly Lys Asp His Pro Gly Gly
   140 145 150 155
   ggc gac cag atc ttc ttc cag ggc cac gca tca cca ggt atg tac gca 2872
   Gly Asp Gln Ile Phe Phe Gln Gly His Ala Ser Pro Gly Met Tyr Ala
   160 165 170
   cgt gca ttc atg gag ggt cgc ctt tct gaa gac gat ctc gat ggc ttc 2920
   Arg Ala Phe Met Glu Gly Arg Leu Ser Glu Asp Asp Leu Asp Gly Phe
   175 180 185
   cgt cag gaa gtt tcc cgt gag cag ggt ggc att ccg tcc tac cct cac 2968
   Arg Gln Glu Val Ser Arg Glu Gln Gly Gly Ile Pro Ser Tyr Pro His
   190 195 200
   cca cac ggt atg aag gac ttc tgg gag ttc cca act gtg tcc atg ggt 3016
   Pro His Gly Met Lys Asp Phe Trp Glu Phe Pro Thr Val Ser Met Gly
   205 210 215
   ctt ggc cca atg gat gcc att tac cag gca cgt ttc aac cgc tac ctc 3064
   Leu Gly Pro Met Asp Ala Ile Tyr Gln Ala Arg Phe Asn Arg Tyr Leu
   220 225 230 235
   gaa aac cgt ggc atc aag gac acc tct gac cag cac gtc tgg gcc ttc 3112
   Glu Asn Arg Gly Ile Lys Asp Thr Ser Asp Gln His Val Trp Ala Phe
   240 245 250
   ctt ggc gac ggc gaa atg gac gag cca gaa tca cgt ggt ctc atc cag 3160
   Leu Gly Asp Gly Glu Met Asp Glu Pro Glu Ser Arg Gly Leu Ile Gln
   255 260 265
   cag gct gca ctg aac aac ctg gac aac ctg acc ttc gtg gtt aac tgc 3208
   Gln Ala Ala Leu Asn Asn Leu Asp Asn Leu Thr Phe Val Val Asn Cys
   270 275 280
   aac ctg cag cgt ctc gac gga cct gtc cgc ggt aac acc aag atc atc 3256
   Asn Leu Gln Arg Leu Asp Gly Pro Val Arg Gly Asn Thr Lys Ile Ile
   285 290 295
   cag gaa ctc gag tcc ttc ttc cgt ggc gca ggc tgg tct gtg atc aag 3304
   Gln Glu Leu Glu Ser Phe Phe Arg Gly Ala Gly Trp Ser Val Ile Lys
   300 305 310 315
   gtt gtt tgg ggt cgc gag tgg gat gaa ctt ctg gag aag gac cag gat 3352
   Val Val Trp Gly Arg Glu Trp Asp Glu Leu Leu Glu Lys Asp Gln Asp
   320 325 330
   ggt gca ctt gtt gag atc atg aac aac acc tcc gat ggt gac tac cag 3400
   Gly Ala Leu Val Glu Ile Met Asn Asn Thr Ser Asp Gly Asp Tyr Gln
   335 340 345
   acc ttc aag gct aac gac ggc gca tat gtt cgt gag cac ttc ttc gga 3448
   Thr Phe Lys Ala Asn Asp Gly Ala Tyr Val Arg Glu His Phe Phe Gly
   350 355 360
   cgt gac cca cgc acc gca aag ctc gtt gag aac atg acc gac gaa gaa 3496
   Arg Asp Pro Arg Thr Ala Lys Leu Val Glu Asn Met Thr Asp Glu Glu
   365 370 375
   atc tgg aag ctg cca cgt ggc ggc cac gat tac cgc aag gtt tac gca 3544
   Ile Trp Lys Leu Pro Arg Gly Gly His Asp Tyr Arg Lys Val Tyr Ala
   380 385 390 395
   gcc tac aag cga gct ctt gag acc aag gat cgc cca acc gtc atc ctt 3592
   Ala Tyr Lys Arg Ala Leu Glu Thr Lys Asp Arg Pro Thr Val Ile Leu
   400 405 410
   gct cac acc att aag ggc tac gga ctc ggc cac aac ttc gaa ggc cgt 3640
   Ala His Thr Ile Lys Gly Tyr Gly Leu Gly His Asn Phe Glu Gly Arg
   415 420 425
   aac gca acc cac cag atg aag aag ctg acg ctt gat gat ctg aag ttg 3688
   Asn Ala Thr His Gln Met Lys Lys Leu Thr Leu Asp Asp Leu Lys Leu
   430 435 440
   ttc cgc gac aag cag ggc atc cca atc acc gat gag cag ctg gag aag 3736
   Phe Arg Asp Lys Gln Gly Ile Pro Ile Thr Asp Glu Gln Leu Glu Lys
   445 450 455
   gat cct tac ctt cct cct tac tac cac cca ggt gaa gac gct cct gaa 3784
   Asp Pro Tyr Leu Pro Pro Tyr Tyr His Pro Gly Glu Asp Ala Pro Glu
   460 465 470 475
   atc aag tac atg aag gaa cgt cgc gca gcg ctc ggt ggc tac ctg cca 3832
   Ile Lys Tyr Met Lys Glu Arg Arg Ala Ala Leu Gly Gly Tyr Leu Pro
   480 485 490
   gag cgt cgt gag aac tac gat cca att cag gtt cca cca ctg gat aag 3880
   Glu Arg Arg Glu Asn Tyr Asp Pro Ile Gln Val Pro Pro Leu Asp Lys
   495 500 505
   ctt cgc tct gtc cgt aag ggc tcc ggc aag cag cag atc gct acc act 3928
   Leu Arg Ser Val Arg Lys Gly Ser Gly Lys Gln Gln Ile Ala Thr Thr
   510 515 520
   atg gcg act gtt cgt acc ttc aag gaa ctg atg cgc gat aag ggc ttg 3976
   Met Ala Thr Val Arg Thr Phe Lys Glu Leu Met Arg Asp Lys Gly Leu
   525 530 535
   gct gat cgc ctt gtc cca atc att cct gat gag gca cgt acc ttc ggt 4024
   Ala Asp Arg Leu Val Pro Ile Ile Pro Asp Glu Ala Arg Thr Phe Gly
   540 545 550 555
   ctt gac tct tgg ttc cca acc ttg aag atc tac aac ccg cac ggt cag 4072
   Leu Asp Ser Trp Phe Pro Thr Leu Lys Ile Tyr Asn Pro His Gly Gln
   560 565 570
   aac tac gtg cct gtt gac cac gac ctg atg ctc tcc tac cgt gag gca 4120
   Asn Tyr Val Pro Val Asp His Asp Leu Met Leu Ser Tyr Arg Glu Ala
   575 580 585
   cct gaa gga cag atc ctg cac gaa ggc atc aac gag gct ggt tcc gtg 4168
   Pro Glu Gly Gln Ile Leu His Glu Gly Ile Asn Glu Ala Gly Ser Val
   590 595 600
   gca tcg ttc atc gct gcg ggt acc tcc tac gcc acc cac ggc aag gcc 4216
   Ala Ser Phe Ile Ala Ala Gly Thr Ser Tyr Ala Thr His Gly Lys Ala
   605 610 615
   atg att ccg ctg tac atc ttc tac tcg atg ttc gga ttc cag cgc acc 4264
   Met Ile Pro Leu Tyr Ile Phe Tyr Ser Met Phe Gly Phe Gln Arg Thr
   620 625 630 635
   ggt gac tcc atc tgg gca gca gcc gat cag atg gca cgt ggc ttc ctc 4312
   Gly Asp Ser Ile Trp Ala Ala Ala Asp Gln Met Ala Arg Gly Phe Leu
   640 645 650
   ttg ggc gct acc gca ggt cgc acc acc ctg acc ggt gaa ggc ctc cag 4360
   Leu Gly Ala Thr Ala Gly Arg Thr Thr Leu Thr Gly Glu Gly Leu Gln
   655 660 665
   cac atg gat gga cac tcc cct gtc ttg gct tcc acc aac gag ggt gtc 4408
   His Met Asp Gly His Ser Pro Val Leu Ala Ser Thr Asn Glu Gly Val
   670 675 680
   gag acc tac gac cca tcc ttt gcg tac gag atc gca cac ctg gtt cac 4456
   Glu Thr Tyr Asp Pro Ser Phe Ala Tyr Glu Ile Ala His Leu Val His
   685 690 695
   cgt ggc atc gac cgc atg tac ggc cca ggc aag ggt gaa gat gtt atc 4504
   Arg Gly Ile Asp Arg Met Tyr Gly Pro Gly Lys Gly Glu Asp Val Ile
   700 705 710 715
   tac tac atc acc atc tac aac gag cca acc cca cag cca gct gag cca 4552
   Tyr Tyr Ile Thr Ile Tyr Asn Glu Pro Thr Pro Gln Pro Ala Glu Pro
   720 725 730
   gaa gga ctg gac gta gaa ggc ctg cac aag ggc atc tac ctc tac tcc 4600
   Glu Gly Leu Asp Val Glu Gly Leu His Lys Gly Ile Tyr Leu Tyr Ser
   735 740 745
   cgc ggt gaa ggc acc ggc cat gag gca aac atc ttg gct tcc ggt gtt 4648
   Arg Gly Glu Gly Thr Gly His Glu Ala Asn Ile Leu Ala Ser Gly Val
   750 755 760
   ggt atg cag tgg gct ctc aag gct gca tcc atc ctt gag gct gac tac 4696
   Gly Met Gln Trp Ala Leu Lys Ala Ala Ser Ile Leu Glu Ala Asp Tyr
   765 770 775
   gga gtt cgt gcc aac att tac tcc gct act tct tgg gtt aac ttg gct 4744
   Gly Val Arg Ala Asn Ile Tyr Ser Ala Thr Ser Trp Val Asn Leu Ala
   780 785 790 795
   cgc gat ggc gct gct cgt aac aag gca cag ctg cgc aac cca ggt gca 4792
   Arg Asp Gly Ala Ala Arg Asn Lys Ala Gln Leu Arg Asn Pro Gly Ala
   800 805 810
   gat gct ggc gag gca ttc gta acc acc cag ctg aag cag acc tcc ggc 4840
   Asp Ala Gly Glu Ala Phe Val Thr Thr Gln Leu Lys Gln Thr Ser Gly
   815 820 825
   cca tac gtt gca gtg tct gac ttc tcc act gat ctg cca aac cag atc 4888
   Pro Tyr Val Ala Val Ser Asp Phe Ser Thr Asp Leu Pro Asn Gln Ile
   830 835 840
   cgt gaa tgg gtc cca ggc gac tac acc gtt ctc ggt gca gat ggc ttc 4936
   Arg Glu Trp Val Pro Gly Asp Tyr Thr Val Leu Gly Ala Asp Gly Phe
   845 850 855
   ggt ttc tct gat acc cgc cca gct gct cgt cgc ttc ttc aac atc gac 4984
   Gly Phe Ser Asp Thr Arg Pro Ala Ala Arg Arg Phe Phe Asn Ile Asp
   860 865 870 875
   gct gag tcc att gtt gtt gca gtg ctg aac tcc ctg gca cgc gaa ggc 5032
   Ala Glu Ser Ile Val Val Ala Val Leu Asn Ser Leu Ala Arg Glu Gly
   880 885 890
   aag atc gac gtc tcc gtt gct gct cag gct gct gag aag ttc aag ttg 5080
   Lys Ile Asp Val Ser Val Ala Ala Gln Ala Ala Glu Lys Phe Lys Leu
   895 900 905
   gat gat cct acg agt gtt tcc gta gat cca aac gct cct gag gaa 5125
   Asp Asp Pro Thr Ser Val Ser Val Asp Pro Asn Ala Pro Glu Glu
   910 915 920
   taaatcacct caagggacag ataaatcccg ccgccagacg ttagtctggc ggcgggattc 5185
   gtcgtaaagc aagctctttt tagccgagaa acgccttgtc agacaatgtt gcgcccttga 5245
   tattggcgaa ctcctgcagc aaatcgcgca cagtcaactt cgacttggta gcctgatctg 5305
   cctggtagac aatctggcct tcatgcatca tgatcaggcg attgcccagg cgaattgcct 5365
   gttcc 5370
<210> 10
   <211> 922
   <212> PRT
   <213> Brevibacterium lactofermentum
<400> 10
   Met Ala Asp Gln Ala Lys Leu Gly Gly Lys Pro Ser Asp Asp Ser Asn
   1 5 10 15
   Phe Ala Met Ile Arg Asp Gly Val Ala Ser Tyr Leu Asn Asp Ser Asp
   20 25 30
   Pro Glu Glu Thr Asn Glu Trp Met Asp Ser Leu Asp Gly Leu Leu Gln
   35 40 45
   Glu Ser Ser Pro Glu Arg Ala Arg Tyr Leu Met Leu Arg Leu Leu Glu
   50 55 60
   Arg Ala Ser Ala Lys Arg Val Ser Leu Pro Pro Met Thr Ser Thr Asp
   65 70 75 80
   Tyr Val Asn Thr Ile Pro Thr Ser Met Glu Pro Glu Phe Pro Gly Asp
   85 90 95
   Glu Glu Met Glu Lys Arg Tyr Arg Arg Trp Ile Arg Trp Asn Ala Ala
   100 105 110
   Ile Met Val His Arg Ala Gln Arg Pro Gly Ile Gly Val Gly Gly His
   115 120 125
   Ile Ser Thr Tyr Ala Gly Ala Ala Pro Leu Tyr Glu Val Gly Phe Asn
   130 135 140
   His Phe Phe Arg Gly Lys Asp His Pro Gly Gly Gly Asp Gln Ile Phe
   145 150 155 160
   Phe Gln Gly His Ala Ser Pro Gly Met Tyr Ala Arg Ala Phe Met Glu
   165 170 175
   Gly Arg Leu Ser Glu Asp Asp Leu Asp Gly Phe Arg Gln Glu Val Ser
   180 185 190
   Arg Glu Gln Gly Gly Ile Pro Ser Tyr Pro His Pro His Gly Met Lys
   195 200 205
   Asp Phe Trp Glu Phe Pro Thr Val Ser Met Gly Leu Gly Pro Met Asp
   210 215 220
   Ala Ile Tyr Gln Ala Arg Phe Asn Arg Tyr Leu Glu Asn Arg Gly Ile
   225 230 235 240
   Lys Asp Thr Ser Asp Gln His Val Trp Ala Phe Leu Gly Asp Gly Glu
   245 250 255
   Met Asp Glu Pro Glu Ser Arg Gly Leu Ile Gln Gln Ala Ala Leu Asn
   260 265 270
   Asn Leu Asp Asn Leu Thr Phe Val Val Asn Cys Asn Leu Gln Arg Leu
   275 280 285
   Asp Gly Pro Val Arg Gly Asn Thr Lys Ile Ile Gln Glu Leu Glu Ser
   290 295 300
   Phe Phe Arg Gly Ala Gly Trp Ser Val Ile Lys Val Val Trp Gly Arg
   305 310 315 320
   Glu Trp Asp Glu Leu Leu Glu Lys Asp Gln Asp Gly Ala Leu Val Glu
   325 330 335
   Ile Met Asn Asn Thr Ser Asp Gly Asp Tyr Gln Thr Phe Lys Ala Asn
   340 345 350
   Asp Gly Ala Tyr Val Arg Glu His Phe Phe Gly Arg Asp Pro Arg Thr
   355 360 365
   Ala Lys Leu Val Glu Asn Met Thr Asp Glu Glu Ile Trp Lys Leu Pro
   370 375 380
   Arg Gly Gly His Asp Tyr Arg Lys Val Tyr Ala Ala Tyr Lys Arg Ala
   385 390 395 400
   Leu Glu Thr Lys Asp Arg Pro Thr Val Ile Leu Ala His Thr Ile Lys
   405 410 415
   Gly Tyr Gly Leu Gly His Asn Phe Glu Gly Arg Asn Ala Thr His Gln
   420 425 430
   Met Lys Lys Leu Thr Leu Asp Asp Leu Lys Leu Phe Arg Asp Lys Gln
   435 440 445
   Gly Ile Pro Ile Thr Asp Glu Gln Leu Glu Lys Asp Pro Tyr Leu Pro
   450 455 460
   Pro Tyr Tyr His Pro Gly Glu Asp Ala Pro Glu Ile Lys Tyr Met Lys
   465 470 475 480
   Glu Arg Arg Ala Ala Leu Gly Gly Tyr Leu Pro Glu Arg Arg Glu Asn
   485 490 495
   Tyr Asp Pro Ile Gln Val Pro Pro Leu Asp Lys Leu Arg Ser Val Arg
   500 505 510
   Lys Gly Ser Gly Lys Gln Gln Ile Ala Thr Thr Met Ala Thr Val Arg
   515 520 525
   Thr Phe Lys Glu Leu Met Arg Asp Lys Gly Leu Ala Asp Arg Leu Val
   530 535 540
   Pro Ile Ile Pro Asp Glu Ala Arg Thr Phe Gly Leu Asp Ser Trp Phe
   545 550 555 560
   Pro Thr Leu Lys Ile Tyr Asn Pro His Gly Gln Asn Tyr Val Pro Val
   565 570 575
   Asp His Asp Leu Met Leu Ser Tyr Arg Glu Ala Pro Glu Gly Gln Ile
   580 585 590
   Leu His Glu Gly Ile Asn Glu Ala Gly Ser Val Ala Ser Phe Ile Ala
   595 600 605
   Ala Gly Thr Ser Tyr Ala Thr His Gly Lys Ala Met Ile Pro Leu Tyr
   610 615 620
   Ile Phe Tyr Ser Met Phe Gly Phe Gln Arg Thr Gly Asp Ser Ile Trp
   625 630 635 640
   Ala Ala Ala Asp Gln Met Ala Arg Gly Phe Leu Leu Gly Ala Thr Ala
   645 650 655
   Gly Arg Thr Thr Leu Thr Gly Glu Gly Leu Gln His Met Asp Gly His
   660 665 670
   Ser Pro Val Leu Ala Ser Thr Asn Glu Gly Val Glu Thr Tyr Asp Pro
   675 680 685
   Ser Phe Ala Tyr Glu Ile Ala His Leu Val His Arg Gly Ile Asp Arg
   690 695 700
   Met Tyr Gly Pro Gly Lys Gly Glu Asp Val Ile Tyr Tyr Ile Thr Ile
   705 710 715 720
   Tyr Asn Glu Pro Thr Pro Gln Pro Ala Glu Pro Glu Gly Leu Asp Val
   725 730 735
   Glu Gly Leu His Lys Gly Ile Tyr Leu Tyr Ser Arg Gly Glu Gly Thr
   740 745 750
   Gly His Glu Ala Asn Ile Leu Ala Ser Gly Val Gly Met Gln Trp Ala
   755 760 765
   Leu Lys Ala Ala Ser Ile Leu Glu Ala Asp Tyr Gly Val Arg Ala Asn
   770 775 780
   Ile Tyr Ser Ala Thr Ser Trp Val Asn Leu Ala Arg Asp Gly Ala Ala
   785 790 795 800
   Arg Asn Lys Ala Gln Leu Arg Asn Pro Gly Ala Asp Ala Gly Glu Ala
   805 810 815
   Phe Val Thr Thr Gln Leu Lys Gln Thr Ser Gly Pro Tyr Val Ala Val
   820 825 830
   Ser Asp Phe Ser Thr Asp Leu Pro Asn Gln Ile Arg Glu Trp Val Pro
   835 840 845
   Gly Asp Tyr Thr Val Leu Gly Ala Asp Gly Phe Gly Phe Ser Asp Thr
   850 855 860
   Arg Pro Ala Ala Arg Arg Phe Phe Asn Ile Asp Ala Glu Ser Ile Val
   865 870 875 880
   Val Ala Val Leu Asn Ser Leu Ala Arg Glu Gly Lys Ile Asp Val Ser
   885 890 895
   Val Ala Ala Gln Ala Ala Glu Lys Phe Lys Leu Asp Asp Pro Thr Ser
   900 905 910
   Val Ser Val Asp Pro Asn Ala Pro Glu Glu
   915 920
<210> 11
   <211> 20
   <212> DNA
   <213> Artificial Sequence
<220>
   <223> Description of Artificial Sequence:primer for constructing plasmid for amplifying pdhA gene of Brevibacterium lactofermentum
<400> 11
   aatgccagga gtcaacaccc 20
<210> 12
   <211> 20
   <212> DNA
   <213> Artificial Sequence
<220>
   <223> Description of Artificial Sequence:primer for constructing plasmid for amplifying pdhA gene of Brevibacterium lactofermentum
<400> 12
   acatggaaca ggcaattcgc 20
<210> 13
   <211> 32
   <212> DNA
   <213> Artificial Sequence
<220>
   <223> Description of Artificial Sequence:primer for amplifying kanamycin resistant gene of Streptococcus faecalis
<400> 13
   cccgttaact gcttgaaacc caggacaata ac 32
<210> 14
   <211> 30
   <212> DNA
   <213> Artificial Sequence
<220>
   <223> Description of Artificial Sequence:primer for amplifying kanamycin resistant gene of Streptococcus faecalis
<400> 14
   cccgttaaca tgtacttcag aaaagattag 30
<210> 15
   <211> 26
   <212> DNA
   <213> Artificial Sequence
<220>
   <223> Description of Artificial Sequence:primer for amplifying Escherichia coli cloning vector pHSG399
<400> 15
   gatatctacg tgccgatcaa cgtctc 26
<210> 16
   <211> 25
   <212> DNA
   <213> Artificial Sequence
<220>
   <223> Description of Artificial Sequence:primer for amplifying Escherichia coli cloning vector pHSG399
<400> 16
   aggccttttt ttaaggcagt tattg 25

## Description

### Technical Field

The present invention relates to a L-glutamic acid-producing bacterium and a method for producing L-glutamic acid by a fermentation method using the same. L-glutamic acid is an important amino acid as a food, medicament or the like.

### Background Art

Heretofore, L-glutamic acid has been produced by a fermentation method using a coryneform L-glutamic acid-producing bacterium belonging to the genus *Brevibacterium, Corynebacterium* (Amino Acid Fermentation, Gakkai Shuppan Center, pp. 195-215, 1986). As such coryneform bacteria, wild strains isolated from nature or mutants thereof are used to improve the producing ability.

Further, there have also been disclosed various techniques for promoting L-glutamic acid-producing ability by enhancing genes for enzymes involved in the L-glutamic acid biosynthetic pathway through recombinant DNA techniques. For example, Japanese Patent Laid-open No. 63-214189 discloses a technique for elevating the L-glutamic acid-producing ability by enhancing a glutamate dehydrogenase gene, isocitrate dehydrogenase gene, aconitate hydratase gene, and citrate synthase gene.

Chemical Abstracts, Vol. 122, No. 17, 24 Apr. 1995 relate to Corynebacterium melassocola metabolic pathways.

Database WPI, Section Ch, Week 198841 discloses genetically modified coryneform bacteria which are used for the production of L-glutamic acid.

US 5 846 790 A, Ajinomoto Co., Inc., 8 Dec. 1998 relates to methods of producing L-lysine and L-glutamic acid by fermentation.

Cole, S.T. et al., Nature, Vol. 393, 11 June 1998) discloses the complete genome sequence of Mycobacterium tubercolosis.

However, it has never been known to utilize a pyruvate dehydrogenase gene for breeding of L-glutamic acid-producing coryneform bacteria.

### Detailed Description of the Invention

An object of the present invention is to breed a bacterial strain which has high L-glutamic acid-producing ability, thereby providing a method for producing L-glutamic acid more efficiently with a lower cost in order to respond to much more increase of the demand of L-glutamic acid.

The inventors of the present invention successfully obtained a coryneform bacterium having improved L-glutamic acid-producing ability by enhancing pyruvate dehydrogenase activity of a coryneform bacterium having L-glutamic acid-producing ability, and accomplished the present invention.

That is, the present invention provides the followings.
(1) A coryneform bacterium which has L-glutamic acid-producing ability and has enhanced intracellular pyruvate dehydrogenase activity.
(2) The coryneform bacterium according (1), wherein the enhanced intracellular pyruvate dehydrogenase activity is obtained by increasing copy number of a gene coding for pyruvate dehydrogenase.
(3) The coryneform bacterium according to (2), wherein the gene coding for pyruvate dehydrogenase is derived from *Escherichia coli*.
(4) A method for producing L-glutamic acid comprising the steps of culturing the coryneform bacterium according to any one of (1) to (3) in a medium to produce and accumulate L-glutamic acid in the medium, and collecting the L-glutamic acid from the medium.
(5) The method according to (4), wherein the medium contains vitamin 81 at 20 µg/L or more.
(6) A DNA which encode a protein defined in the following (A) or (B):
   (A) a protein which has the amino acid sequence of SEQ ID NO: 10;
   (B) a protein which has the amino acid sequence of SEQ ID NO: 10 including substitution, deletion, insertion, addition, or inversion of one or several amino acids, and has a pyruvate dehydrogenase activity.
(7) The DNA of (6) which is a DNA defined in the following (a) or (b):
   (a) a DNA which comprises the nucleotide sequence of nucleotide numbers 2360 to 5120 in SEQ ID NO: 9;
   (b) a DNA which is hybridizable with a nucleotide sequence of nucleotide numbers 2360 to 5120 in SEQ ID NO: 9 or a probe prepared from the nucleotide sequence under a stringent condition, and encodes for a protein having a pyruvate dehydrogenase activity.
(8) The DNA of (7) wherein the stringent condition is a condition in which washing is performed at 60°C, and at a salt concentration corresponding to 1 x SSC and 0.1 % SDS.

The term "L-glutamic acid-producing ability" used for the present invention means an ability of the coryneform bacterium of the present invention to accumulate L-glutamic acid in a medium when the bacterium is cultured in the medium. This L-glutamic acid-producing ability may either be a property possessed by a wild-type strain of coryneform bacteria, or a property imparted or enhanced by breeding.

Hereafter, the present invention will be explained in detail.

### <1> Coryneform bacterium of the present invention

The coryneform bacterium of the present invention is a coryneform bacterium having enhanced intracellular pyruvate dehydrogenase activity, and L-glutamic acid-producing ability.

The coryneform bacteria includes bacteria having been hitherto classified into the genus *Brevibacterium* but united into the genus *Corynebacterium* at present (Int. J. Syst. Bacteriol., 41, 255 (1981)), and include bacteria belonging to the genus *Brevibacterium* closely relative to the genus *Corynebacterium*. Examples of such coryneform L-glutamic acid-producing bacteria include the followings.
*Corynebacterium acetoacidophilum*
*Corynebacterium acetoglutamicum*
*Corynebacterium alkanolyticum*
*Corynebacterium callunae*
*Corynebacterium glutamicum*
*Corynebacterium lilium* (*Corynebacterium glutamicum*)
*Corynebacterium melassecola*
*Corynebacterium thermoaminogenes*
*Corynebacterium herculis*
*Brevibacterium divaricatum* (*Corynebacterium glutamicum*)
*Brevibacterium flavum* (*Corynebacterium glutamicum*)
*Brevibacterium immariophilum*
*Brevibacterium lactofermentum* (*Corynebacterium glutamicum*)
*Brevibacterium roseum*
*Brevibacterium saccharolyticum*
*Brevibacterium thiogenitalis*
*Brevibacterium ammoniagenes* (*Corynebacterium ammoniagenes*)
*Brevibacterium album*
*Brevibacterium cerinum*
*Microbacterium ammoniaphilum*

Specifically, the following strains of these bacteria are exemplified:
*Corynebacterium acetoacidophilum* ATCC13870
*Corynebacterium acetoglutamicum* ATCC15806
*Corynebacterium alkanolyticum* ATCC21511
*Corynebacterium callunae* ATCC15991
*Corynebacterium glutamicum* ATCC13020, 13032, 13060
*Corynebacterium lilium* (*Corynebacterium glutamicum*) ATCC15990
*Corynebacterium melassecola* ATCC17965
*Corynebacterium thermoaminogenes* AJ12340 FERM BP-1539)
*Corynebacterium herculis* ATCC13868
*Brevibacterium divaricatum* (*Corynebacterium glutamicum*) ATCC14020
*Brevibacterium flavum* (*Corynebacterium glutamicum*) ATCC13826, ATCC14067
*Brevibacterium immariophilum* ATCC14068
*Brevibacterium lactofermentum* (*Corynebacterium glutamicum*) ATCC13665, ATCC13869
*Brevibacterium roseum* ATCC13825
*Brevibacterium saccharolyticum* ATCC14066
*Brevibacterium thiogenitalis* ATCC19240
*Corynebacterium ammoniagenes* (*Brevibacterium ammoniagenes*) ATCC6871
*Brevibacterium album* ATCC15111
*Brevibacterium cerinum* ATCC15112
*Microbacterium ammoniaphilum* ATCC15354

These strains can be provided from, for example, the American Type Culture Collection. Each strain is assigned its registration number, and one can request provision of each strain by referring to its registration number. The registration numbers corresponding to the strains are indicated on the catalog of the American Type Culture Collection (Address: 12301 Parklawn Drive, Rockville, Maryland 20852, United States of America). The AJ12340 strain was deposited at the National Institute of Bioscience and Human-Technology, Agency of Industrial Science and Technology, Ministry of International Trade and Industry under the provisions of the Budapest Treaty, and received an accession number of FERM BP-1539.

### <2> Amplification of pyruvate dehydrogenase activity

In order to amplify the pyruvate dehydrogenase activity in a coryneform bacterial cell, a recombinant DNA can be prepared by ligating a gene fragment coding for pyruvate dehydrogenase with a vector functioning in the bacterium, preferably a multi-copy vector, and introduced into the host bacterial cell having L-glutamic acid-producing ability to transform the cell. The copy number of the gene coding for pyruvate dehydrogenase in the cell of the transformant strain is thereby increased, and as a result, the pyruvate dehydrogenase activity is amplified.

In *Escherichia coli*, the pyruvate dehydrogenase is present as a heterooligomer consisting of three subunits, E1, E2, and E3, which are encoded by *aceE* gene, *aceF* gene, and *1pd* gene, respectively, and these genes form an operon. The term "gene coding for pyruvate dehydrogenase" or "pyruvate dehydrogenase gene" used for the present invention means an operon that codes for such a pyruvate dehydrogenase complex, or a gene which codes for a monomer having the pyruvate dehydrogenase activity. An example of the monomer having the pyruvate dehydrogenase activity is the E1 subunit encoded by the *pdhA* gene of *Brevibacterium lactofermentum.*

As the pyruvate dehydrogenase gene, those derived form coryneform bacteria as well as those derived from other organisms such as *Escherichia* bacteria may be used.

The nucleotide sequence of the pyruvate dehydrogenase complex gene of *Escherichia coli* K-12 has already been elucidated (FEMS Microbiology Letters, Vol. 44, p.417, 1987). Therefore, the pyruvate dehydrogenase gene can be obtained by PCR (polymerase chain reaction; see White, T.J. et al; Trends Genet. 5, 185 (1989)) utilizing primers produced based on the nucleotide sequence, for example, the primers represented in Sequence Listing as SEQ ID NOS: 1 and 2, and *Escherichia coli* chromosome DNA as a template. The pyruvate dehydrogenase genes derived from other microorganisms may be obtained similarly. The preparation of the gene derived from coryneform bacteria will be explained later.

The chromosome DNA can be prepared from a bacterium, which is a DNA donor, by the method of Saito and Miura (H. Saito and K. Miura, Biochem. Biophys. Acta, 72, 619 (1963)), for example.

The pyruvate dehydrogenase gene amplified by PCR is preferably ligated to a vector DNA autonomously replicable in a cell of *Escherichia coli* and/or coryneform bacteria to form a recombinant DNA. By introducing this recombinant DNA into an *Escherichia coli* cell, the subsequent procedure can be made easy. The vector autonomously replicable in *Escherichia coli* cells is preferably a plasmid vector autonomously replicable in the host cell, and examples thereof include pUC19, pUC18, pBR322, pHSG299, pHSG399, pHSG398, RSF1010 and so forth.

The plasmids which function in coryneform bacteria means plasmids autonomously replicable in coryneform bacteria. The plasmids includes, for example, the followings.
pAM330 (see Japanese Patent Laid-open No. 58-67699)
pHM1519 (see Japanese Patent Laid-open No. 58-77895)
pAJ655 (see Japanese Patent Laid-open No. 58-192900)
pAJ611 (see the same)
pAJ1844 (see the same)
pCG1 (see Japanese Patent Laid-open No. 57-134500)
pCG2 (see Japanese Patent Laid-open No. 58-35197)
pCG4 (see Japanese Patent Laid-open No. 57-183799)
pCG11 (see the same)
pHK4 (see Japanese Patent Laid-open No. 5-7491)

In order to prepare recombinant DNA by ligating the pyruvate dehydrogenase gene and a vector which can function in a cell of coryneform bacterium, the vector is digested by restriction enzyme(s) corresponding to the termini of the pyruvate dehydrogenase gene. Ligation is generally performed by using a ligase such as T4 DNA ligase.

To introduce the recombinant DNA prepared as described above to a coryneform bacterium, any known transformation methods can be employed. For instance, employable are a method of treating recipient cells with calcium chloride so as to increase the permeability of DNA, which has been reported for *Escherichia coli* K-12 [see Mandel, M. and Higa, A., J. Mol. Biol., 53, 159 (1970)]; and a method of preparing competent cells from cells which are at the growth phase followed by introducing the DNA thereinto, which has been reported for *Bacillus subtilis* [see Duncan, C.H., Wilson, G.A. and Young, F.E., Gene, 1, 153 (1977)]. In addition to these, also employable is a method of making DNA-recipient cells into the protoplast or spheroplast which can easily take up recombinant DNAs followed by introducing the recombinant DNA into the cells, which is known to be applicable to *Bacillus subtilis,* actinomycetes and yeasts [see Chang, S. and Choen, S.N., Molec. Gen. Genet., 168, 111 (1979); Bibb, M.J., Ward, J.M. and Hopwood, O.A., Nature, 274, 398 (1978); Hinnen, A., Hicks, J.B. and Fink, G.R., Proc. Natl. Sci., USA, 75, 1929 (1978)]. The method of transformation used in embodiments of the present invention is the electric pulse method (refer to Japanese Patent Publication Laid-Open No. 2-207791).

Enhancement of pyruvate dehydrogenase activity can also be achieved by introducing multiple copies of the pyruvate dehydrogenase gene into the chromosomal DNA of coryneform bacterium. In order to introduce multiple copies of the pyruvate dehydrogenase gene in the chromosomal DNA of corynefrom bacterium, the homologous recombination is carried out using a sequence whose multiple copies exist in the chromosomal DNA as targets. As sequences whose multiple copies exist in the chromosomal DNA, repetitive DNA, inverted repeats exist at the end of a transposable element can be used. Also, as disclosed in Japanese Patent Laid-open No. 2-109985, it is possible to incorporate the pyruvate dehydrogenase gene into transposon, and allow it to be transferred to introduce multiple copies of the pyruvate dehydrogenase gene into the chromosomal DNA. By either method, the number of copies of the pyruvate dehydrogenase gene within cells of the transformant strain increases, and as a result, pyruvate dehydrogenase activity is enhanced. Enhancement of the expression regulatory sequences may be combined with increasing copy number of the pyruvate dehydrogenase gene.

The enhancement of pyruvate dehydrogenase activity can also be obtained by, besides being based on the aforementioned gene enhancement, enhancing an expression regulatory sequence for the pyruvate dehydrogenase gene. Specifically, it can be attained by replacing an expression regulatory sequence of pyruvate dehydrogenase gene on chromosome DNA or plasmid, such as a promoter, with a stronger one (see Japanese Patent Laid-open No. 1-215280). For example, lac promoter, trc promoter, tac promoter, PR promoter and PL promoter of lambda phage and the like are known as strong promoters.
Substitution of these promoters enhances expression of the pyruvate dehydrogenase gene, and hence the pyruvate dehydrogenase activity is enhanced.

In the coryneform bacteria of the present invention, the activity of an enzyme which catalyzes the biosynthesis of L-glutamic acid other than pyruvate dehydrogenase may be enhancing. Illustrative examples of the enzyme for catalyzing the biosynthesis of L-glutamic acid include glutamate dehydrogenase, glutamine synthetase, glutamate synthase, isocitrate dehydrogenase, aconitate hydratase, citrate synthase, phosphoenolpyruvate carboxylase, pyruvate kinase, enolase, phosphoglyceromutase, phosphoglycerate kinase, glyceraldehyde-3-phosphate dehydrogenase, triosephosphate isomerase, fructose bisphosphate aldolase, phosphofructokinase, glucose phosphate isomerase and the like.

The activity of an enzyme which catalyzes a reaction for generating a compound other than L-glutamic acid by branching off from the biosynthetic pathway of L-glutamic acid may be decreased or lost. Illustrative examples of the enzyme which catalyzes a reaction for generating a compound other than L-glutamic acid by branching off from the biosynthetic pathway of L-glutamic acid include a-ketoglutarate dehydrogenase (aKGDH), isocitrate lyase, phosphate acetyltransferase, acetate kinase, acetohydroximate synthase, acetolactate synthase, formate acetyltransferase, lactate dehydrogenase, glutamate decarboxylase, 1-pyrroline dehydrogenase and the like. Out of these enzymes, aKGDH is preferred.

Furthermore, by introducing a thermosensitive mutation for a biotin activity-inhibiting substance such as surface active agents into a coryneform bacterium having L-glutamic acid-producing ability, the bacterium becomes to be able to produce L-glutamic acid in a medium containing an excessive amount of biotin in the absence of a biotin activity inhibiting substance (see WO96/06180). As such a coryneform bacterium, the *Brevibacterium lactofermentum* AJ13029 strain disclosed in WO96/06180 can be mentioned. The AJ13029 strain was deposited at the National Institute of Bioscience and Human-Technology, Agency of Industrial Science and Technology on September 2, 1994, and received an accession number of FERM P-14501. Then, its was transferred to an international deposition under the provisions of the Budapest Treaty on August 1, 1995, and received an accession number of FERM BP-5189.

### <3> Pyruvate dehydrogenase gene of Brevibacterium lactofermentum

Pyruvate dehydrogenase gene of *Brevibacterium lactofermentum* (*pdhA*) can be obtained through PCR utilizing chromosome DNA of *Brevibacterium lactofermentum*, for example, *Brevibacterium lactofermentum* ATCC13869, as a template, as well as a primer having the nucleotide sequence of SEQ ID NO: 11 and a primer having the nucleotide sequence of SEQ ID NO: 12.

While the DNA of the present invention was obtained by PCR as described above, it can also be obtained from a chromosome DNA library of *Brevibacterium lactofermentum* by hybridization utilizing a probe composed of an oligonucleotide prepared based on the nucleotide sequence of the DNA of the present invention.

Methods for construction of genomic DNA library, hybridization, PCR, preparation of plasmid DNA, digestion and ligation of DNA, transformation and the like are described in Sambrook, J., Fritsch, and E.F., Maniatis, T., Molecular Cloning, Cold Spring Harbor Laboratory Press, 1.21 (1989).

The DNA of the present invention may code for pyruvate dehydrogenase including substitution, deletion, insertion, addition, or inversion of one or several amino acids at one or a plurality of positions, provided that the activity of pyruvate dehydrogenase encoded thereby is not deteriorated. Although the number of "several" amino acids differs depending on the position or the type of amino acid residues in the three-dimensional structure of the protein, it may be 2 to 400, preferably 2 to 100, and more preferably 2 to 20.

DNA, which codes for the substantially same protein as pyruvate dehydrogenase as described above, is obtained, for example, by modifying the nucleotide sequence of pyruvate dehydrogenase gene, for example, by means of the site-directed mutagenesis method so that one or more amino acid residues at a specified site of the gene involve substitution, deletion, insertion, addition, or inversion. DNA modified as described above may be obtained by the conventionally known mutation treatment. The mutation treatment includes a method for treating DNA coding for pyruvate dehydrogenase in vitro, for example, with hydroxylamine, and a method for treating a microorganism, for example, a bacterium belonging to the genus Escherichia harboring DNA coding for pyruvate dehydrogenase with ultraviolet irradiation or a mutating agent such as N-methyl-N'-nitro-N-nitrosoguanidine (NTG) and nitrous acid usually used for the mutation treatment.

The substitution, deletion, insertion, addition, or inversion of nucleotide as described above also includes mutation (mutant or variant) which naturally occurs, for example, the difference in strains, species or genera of the microorganism.

The DNA, which codes for substantially the same protein as pyruvate dehydrogenase, is obtained by expressing DNA having mutation as described above in an appropriate cell, and investigating the pyruvate dehydrogenase activity of an expressed product. The DNA, which codes for substantially the same protein as pyruvate dehydrogenase, is also obtained by isolating DNA which is hybridizable with DNA having, for example, the nucleotide sequence of nucleotide numbers 2360 to 5125 in SEQ ID NO: 9 or a probe which can be prepared from the DNA, under a stringent condition, and which codes for a protein having the activity pyruvate dehydrogenaseto, from DNA coding for pyruvate dehydrogenase having mutation or from a cell harboring it. The probe can be prepared by amplifying suitable region from the sequence of SEQ ID NO: 9 by means of PCR. The "stringent condition" referred to herein is a condition under which so-called specific hybrid is formed, and non-specific hybrid is not formed. It is difficult to clearly express this condition by using any numerical value. However, for example, the stringent condition includes a condition under which DNA's having high homology, for example, DNA's having homology of not less than 40% are hybridized with each other, and DNA's having homology lower than the above are not hybridized with each other. Alternatively, the stringent condition is exemplified by a condition under which DNA's are hybridized with each other at a salt concentration corresponding to an ordinary condition of washing in Southern hybridization, i.e., 60° C, 1 x SSC, 0.1% SDS, preferably 0.1 x SSC, 0.1% SDS.

The gene, which is hybridizable under the condition as described above, includes those having a stop codon generated in the gene, and those having no activity due to mutation of active center. However, such mutant genes can be easily removed by ligating the gene with a commercially available activity expression vector, and measuring the pyruvate dehydrogenase activity in accordance with known method (Methods in Enzymology, 9, 248-249 (1966)).

### <4> Production of L-glutamic acid using coryneform bacterium of the present invention

Production of L-glutamic acid using a bacterial strain belonging to coryneform bacteria and having amplified pyruvate dehydrogenase activity and L-glutamic acid-producing ability can be performed in a conventional manner using a conventional nutrient medium containing a carbon source, nitrogen source, and mineral salts as well as organic trace nutrients such as amino acids and vitamins, as required. Either a synthetic medium or a natural medium may be used. Any kinds of carbon source and nitrogen source may be used so long as they can be utilized by a strain to be cultured.

As the carbon source, it is possible to use sugars such as glucose, glycerol, fructose, sucrose, maltose, mannose galactose, starch hydrolysate and molasses or the like; or organic acids such as acetic acid, citric acid and succinic acid or the like, which can be used solely or combining each other.

As the nitrogen source, it is possible to use ammonia, ammonium sulfate, ammonium carbonate, ammonium chloride, ammonium phosphate or ammonium acetate or the like.

As the organic trace nutrients, amino acids, vitamins, fatty acids, nucleic acids, those containing those substances such as peptone, casamino acid, yeast extract, and soybean protein decomposition product and the like are used. When an auxotrophic mutant that requires an amino acid and so forth for its growth is used, the required nutrient must be supplemented.

As the mineral salts, phosphates, magnesium salts, calcium salts, iron salts, manganese salts and so forth are used.

The coryneform bacterium of the present invention can produce L-glutamic acid of an amount comparable to that obtained in a conventional method even in the presence of vitamin B₁ in an amount smaller than that required for the culture of conventional L-glutamic acid-producing bacteria, for example, 20 µg/L or less of vitamin B₁. On the other hand, the production amount of L-glutamic acid can be increased by adding 20 µg/L or more, preferably 500 µg/L or more of vitamin B₁ to a medium used for the culture of the coryneform bacterium of the present invention.

The culture is performed as aeration culture, while the fermentation temperature is controlled to be 20-45°C, and pH to be 5-9. When pH falls during the culture, the medium is neutralized by addition of calcium carbonate or with an alkali such as ammonia gas. A substantial amount of L-glutamic acid is accumulated in the culture broth after 10 hours to 4 days of culture in such a manner as described above.

Collection of L-glutamic acid from the culture broth after the culture may be performed in a conventional manner. For example, after the cells were removed from the culture broth, L-glutamic acid may be collected by concentrating the broth to crystallize it or by ion exchange chromatography or the like.

### Brief explanation of the Drawings

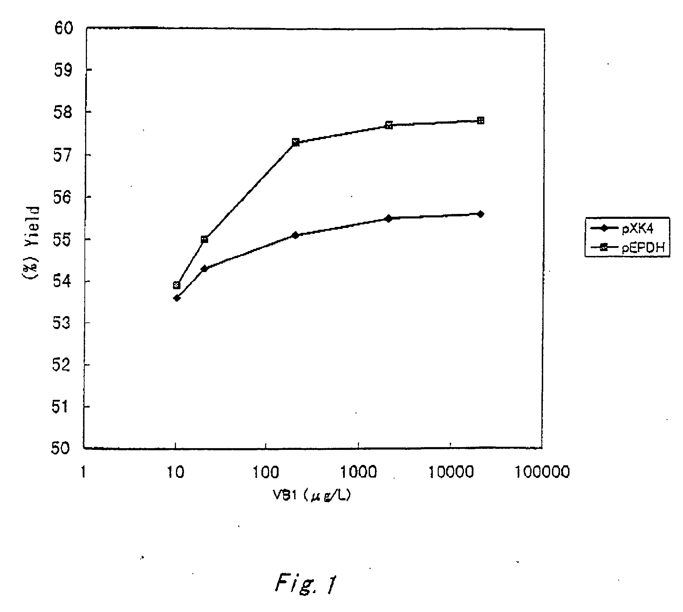

Fig. 1 shows effect of vitamin B₁ on the L-glutamic acid-producing ability of coryneform bacteria.
Fig. 2 shows construction of the plasmid pK1.
Fig. 3 shows construction of the plasmid pSFK6.

### Best Mode for Carrying out the Invention

Hereafter, the present invention will be explained more specifically with reference to the following examples.

### Example 1

### <1> Cloning of pyruvate dehydrogenase gene of Escherichia coli K-12

The nucleotide sequence of the pyruvate dehydrogenase gene of *Escherichia coli* K-12 has already been elucidated (FEMS Microbiology Letters, Vol. 44, p.417, 1987). Primers represented as SEQ ID NOS: 1 and 2 in Sequence Listing were synthesized based on the reported nucleotide sequence, and the pyruvate dehydrogenase gene was amplified by PCR using chromosome DNA of JM109 strain derived from *Escherichia coli* K-12 (produced by Takara Shuzo Co., Ltd.) as a template.

Among the synthesized primers, SEQ ID NO: 1 basically corresponds to the sequence of the 1039th to the 1071st nucleotides of the nucleotide sequence of the pyruvate dehydrogenase gene described in FEMS Microbiology Letters, Vol. 44, p.417, 1987, but it comprises substitutions of A for the 1039th, 1042nd and 1046th nucleotides, substitutions of T for the 1040th and 1043rd nucleotides, substitution of G for the 1044th nucleotide, and insertion of a recognition sequence for restriction enzyme *Xho*I. SEQ ID NO: 2 basically corresponds to a complimentary strand of the sequence of the 7700th to the 7732nd nucleotides of the nucleotide sequence of the pyruvate dehydrogenase gene described in FEMS Microbiology Letters, Vol. 44, p.417, 1987, but it comprises substitutions of A for the 7726th, 7727th and 7730th nucleotides, substitution of C for the 7729th nucleotide, substitution of T for the 7724th nucleotide, and insertion of a recognition sequence for restriction enzyme *Xho*I, and it is represented from its 5' end.

The chromosome DNA of *Escherichia coli* JM109 strain was prepared in a conventional manner (Text for Bioengineering Experiments, Edited by the Society for Bioscience and Bioengineering, Japan, pp.97-98, Baifukan, 1992). For the PCR, standard reaction conditions mentioned in page 185 of "PCR-ho Saizensen (Front Line of PCR)" (Ed. by Takeo Sekiya et al., Kyoritu Shuppan Co., Ltd., 1989) were used.

The obtained PCR product was purified in a conventional manner, treated with restriction enzymes *Xba*I and *Xho*I, ligated to pHSG396 (produced by Takara Shuzo Co., Ltd.) digested with restriction enzymes *Xba*I and *Xho*I by using a ligation kit (produced by Takara Shuzo Co., Ltd.), and used for transformation of competent cells of *Escherichia coli* JM109 (produced by Takara Shuzo Co., Ltd.). The cells were applied on L medium (10 g/L of Bacto trypton, 5 g/L of Bacto yeast extract, 5 g/L of NaCl, and 15 g/L of agar, pH 7.2) containing 10 µg/ml of IPTG (isopropyl-β-D-thiogalactopyranoside), 40 µg/ml of X-Gal (5-bromo-4-chloro-3-indolyl-β-D-galactoside) and 20 µg/ml of chloramphenicol, and cultured overnight. Then, the formed white colonies were picked up, and subjected to single colony isolation to obtain transformant strains.

Plasmids were prepared from the transformants by using the alkaline method (Text for Bioengineering Experiments, Edited by the Society for Bioscience and Bioengineering, Japan, p.105, Baifukan, 1992), and restriction maps of DNA fragments inserted into the vectors were prepared and compared with the reported restriction map of the pyruvate dehydrogenase gene (FEMS Microbiology Letters, Vol. 44, p.417, 1987). A plasmid comprising an inserted DNA fragment having the same restriction map was designated as pHSGEPDH.

The prepared plasmid pHSGEPDH was treated with restriction enzymes *Xba*I and *Sac*I, ligated to pMW219 (produced by Nippon Gene) digested with restriction enzymes *Xba*I and *Sac*I by using a ligation kit (produced by Takara Shuzo Co., Ltd.), and used for transformation of competent cells of *Escherichia coli* JM109 (produced by Takara Shuzo Co., Ltd.). The cells were applied on L medium (10 g/L of Bacto trypton, 5 g/L of Bacto yeast extract, 5 g/L of NaCl, and 15 g/L of agar, pH 7.2) containing 10 µg/ml of IPTG (isopropyl-β-D-thiogalactopyranoside), 40 µg/ml of X-Gal (5-bromo-4-chloro-3-indolyl-β-D-galactoside) and 25 µg/ml of kanamycin, and cultured overnight. Then, the formed white colonies were picked up, and subjected to single colony isolation to obtain transformants. Plasmids were prepared from the transformants by using the alkaline method (Text for Bioengineering Experiments, Edited by the Society for Bioscience and Bioengineering, Japan, p.105, Baifukan, 1992), and restriction maps of DNA fragments inserted into the vectors were prepared and compared with the reported restriction map of the pyruvate dehydrogenase gene. A plasmid comprising an inserted DNA fragment having the same restriction map was designated as pMWEPDH.

Further, in order to confirm expression of the cloned pyruvate dehydrogenase gene, the pyruvate dehydrogenase activity of the JM109 strain and the JM109 strain harboring pMWEPDH was measured by the method of Willms et al. (Methods in Enzymology, Vol. 9, pp.248-249, 1966). As a result, the JM109 strain harboring pMWEPDH showed the pyruvate dehydrogenase activity about four times higher than that of the JM109 strain as shown in Table 1, and thus the expression of the pyruvate dehydrogenase gene was confirmed.

**Table 1**

| Strain | PDH activity (units/mg protein) |
|---|---|
| JM109 | 0.03 |
| JM109 / pMWEPDH | 0.12 |

### <2> Construction of plasmid containing pyruvate dehydrogenase gene of JM109 derived from Escherichia coli K-12 and replication origin of coryneform bacterium

In order to construct a plasmid containing the pyruvate dehydrogenase gene of the *Escherichia coli* JM109 and a replication origin of coryneform bacterium, a gene fragment containing a replication origin was prepared by digesting the plasmid pHK4 (Japanese Patent Laid-open No. 5-7491) having a replication origin derived from the already obtained plasmid pHM1519 autonomously replicable in coryneform bacteria (Agric. Biol. Chem., 48, 2901-2903 (1984)) with restriction enzymes *Bam*HI and *Kpn*I, blunt-ended by using a DNA blunting kit (produced by Takara Shuzo Co., Ltd., Blunting kit), and inserted into the *Xba*I site of the plasmid pMWEPDH containing the pyruvate dehydrogenase gene of the *Escherichia coli* JM109 strain cloned in the above <1> using an *Xba*I linker (produced by Takara Shuzo Co., Ltd.). This plasmid was designated as pEPDH.

### <3> Introduction of pEPDH into coryneform bacteria and evaluation of culture

The *Brevibacterium lactofermentum* AJ13029 strain was transformed with the plasmid pEPDH by the electric pulse method (see Japanese Patent Laid-open No. 2-207791) to obtain a transformants. The obtained transformants AJ13029/pEPDH was cultured for L-glutamic acid production as follows. Cells of the AJ13029/pEPDH strain obtained by culture on a CM2B plate medium containing 25 µg/mL of kanamycin were inoculated to a medium containing 30 g of glucose, 1 g of KH₂PO₄, 0.4 g of MgSO₄·7H₂O, 30 g of (NH₄)₂SO₄, 0.01 g of FeSO₄·7H₂O, 0.01 g of MnSO₄·7H₂O, 15 ml of soybean hydrolysate, 200 µg of thiamin hydrochloride, 60 µg of biotin, 25 mg of kanamycin, and 50 g of CaCO₃ in 1 L of pure water (adjusted to pH 8.0 with KOH), and cultured at 31.5°C with shaking until sugars in the medium was consumed. The obtained culture was inoculated to a medium having the same composition as mentioned above in an amount of 5%, and cultured at 37°C with shaking until sugars in the medium was consumed. As a control, the *Brevibacterium lactofermentum* AJ13029 strain transformed with pHK4 by the electric pulse method was cultured in the same manner as described above.

After the culture was completed, the amount of L-glutamic acid accumulated in the medium was measured by Biotech Analyzer AS-210 produced by Asahi Chemical Industry Co., Ltd. The results are shown in Table 2.

The *Brevibacterium lactofermentum* AJ13029 strain was deposited at the National Institute of Bioscience and Human-Technology, Agency of Industrial Science and Technology, Ministry of International Trade and Industry, on September 2, 1994, and received an accession number of FERM P-14501. Then, its was transferred to an international deposition under the provisions of the Budapest Treaty on August 1, 1995, and received an accession number of FERM BP-5189.

**Table 2**

| Strain | Yield of L-glutamic acid (%) |
|---|---|
| AJ13029 / pHK4 | 55.1 |
| AJ13029 / pEPDH | 57.3 |

### <4> Evaluation of effect of vitamin B₁ addition on L-glutamic acid-producing ability

In order to investigate effect of addition of vitamin B₁ (thiamin), which is a precursor for a coenzyme of the pyruvate dehydrogenase, thiamin diphosphate (TPP), the strain AJ13029/pEPDH having enhanced pyruvate dehydrogenase activity was cultured for L-glutamic acid production in media containing vitamin B₁ at various concentrations as follows.

Cells of the AJ13029/pEPDH strain obtained by culture on a CM2B plate medium containing 25 µg/mL of kanamycin were inoculated to a medium containing 30 g of glucose, 1 g of KH₂PO₄, 0.4 g of MgSO₄·7H₂O, 30 g of (NH₄)₂SO₄, 0.01 g of FeSO₄·7H₂O, 0.01 g of MnSO₄·7H₂O, 15 ml of soybean hydrolysate, 60 µg of biotin, 25 mg of kanamycin, and 50 g of CaCO₃ in 1 L of pure water (adjusted to pH 8.0 with KOH), and cultured at 31.5°C with shaking until the sugars in the medium was consumed. The obtained culture was inoculated in an amount of 5% to a medium having the same composition as above or the same medium containing 10 µg, 20 µg, 200 µg, 2 mg or 20 mg of thiamin hydrochloride, and cultured at 37°C with shaking until the saccharide in the medium was consumed. As a control, the *Brevibacterium lactofermentum* AJ13029 strain transformed with pHK4 by the electric pulse method (see Japanese Patent Laid-open No. 2-207791) was cultured in the same manner as described above. After the culture was completed, the amount of L-glutamic acid accumulated in the medium was measured by Biotech Analyzer AS-210 produced by Asahi Chemical Industry Co., Ltd. The results are shown in Table 3.

**Table 3**

| Strain | Thiamin hydrochloride concentration (µg/L) | Yield of L-glutamic acid (%) |
|---|---|---|
| AJ13029/pHK4 | 0 | 53.5 |
| | 10 | 53.6 |
| | 20 | 54.3 |
| | 200 | 55.1 |
| | 2000 | 55.5 |
| | 20000 | 55.6 |
| AJ13029/pEPDH | 0 | 53.6 |
| | 10 | 53.9 |
| | 20 | 55.0 |
| | 200 | 57.3 |
| | 2000 | 57.7 |
| | 20000 | 57.8 |

From the above results, it was found that the L-glutamic acid-producing bacteria belonging to coryneform bacteria whose pyruvate dehydrogenase activity was enhanced by introduction of the pyruvate dehydrogenase gene showed improved yield of L-glutamic acid.

Moreover, it was also found that the yield is improved when the bacteria were cultured in a medium containing vitamin B. at a concentration of 20 µg/L or higher for both of the L-glutamic acid-producing bacteria belonging to coryneform bacteria and the L-glutamic acid-producing bacteria belonging to coryneform bacteria whose pyruvate dehydrogenase activity was enhanced by introduction of the pyruvate dehydrogenase gene.

### Example 2

### <1> Cloning of pdhA gene derived from Brevibacterium lactofermentum ATCC13869

Among the E1 subunits of pyruvate dehydrogenases of *Escherichia coli*, *Pseudomonas* aeruginosa, and *Mycobacterium tuberculosis*, regions exhibiting high homology were selected, and primers shown in SEQ ID NO: 3 and SEQ ID NO: 4 were synthesized based on the regions. PCR was performed by using these primers and chromosome of *Brevibacterium lactofermentum* ATCC13869 prepared by Bacterial Genomic DNA Purification Kit produced by Advanced Genetic Technologies Corp. as a template under the standard reaction conditions described in "PCR Technology", page 8, Ed. by Henry Ehrlich, Stockton Press, 1989. The reaction solution was subjected to agarose gel electrophoresis, and it was found that a DNA fragment of about 1.3 kb was amplified.

The obtained DNA was sequenced for the nucleotide sequences of the both ends by using the synthetic DNA of SEQ ID NO: 3 and SEQ ID NO: 4. The nucleotide sequencing was performed according to the method of Sanger (J. Mol. Biol., 143, 161 (1980)) by using DNA Sequencing Kit (produced by Applied Biosystems Co.). The determined nucleotide sequence was translated into an amino acid sequence, and compared with E1 subunits of pyruvate dehydrogenases of *Escherichia coli, Pseudomonas aeruginosa*, and *Mycobacterium tuberculosis.* As a result, high homology was observed. Therefore, it was judged that the DNA fragment amplified by the PCR should be a part of the *pdhA* gene coding for the E1 subunit of pyruvate dehydrogenase of *Brevibacterium lactofermentum* ATCC13869, and its upstream region and downstream region were cloned as follows.

DNA fragments were obtained by digestion of *Brevibacterium lactofermentum* ATCC13869 chromosome with restriction enzymes *Eco*RI, *Bam*HI, *Hind*III, *Pst*I, *Sal*I, and *Xba*I (produced by Takara Shuzo Co., Ltd.), and their upstream regions and downstream regions were cloned. The cloning was performed by using primers shown as SEQ ID NO: 5 and SEQ ID NO: 6 in Sequence Listing for the cloning of the upstream regions, and primers shown as SEQ ID NO: 7 and SEQ ID NO: 8 for the cloning of the downstream regions, and using LA PCR in vitro cloning kit (produced by Takara Shuzo Co., Ltd.). As a result of the PCR utilizing the kit, DNA fragments of about 0.5, 2.5, 3.0, 1.5 and 1.8 kb were amplified from the upstream regions digested with *Eco*RI, *Hind*III, *Pst*I, *Sal*I and *Xba*I, respectively, and DNA fragments of about 1.5, 1.0 and 3.5 kb were amplified from the downstream regions digested with *Bam*HI, *Hind*III and *Pst*I, respectively. Therefore, nucleotide sequences of these DNA fragments were determined.

As a result, it was found that an open reading frame for about 920 amino acids was further contained in the amplified DNA fragments, and a region considered to be a promoter region was also present in the upstream of it. Because the amino acid sequence of the product deduced from the nucleotide sequence of the open reading frame showed high homology with the known pyruvate dehydrogenase E1 subunits of *Escherichia coli* and the like, the open reading frame was estimated to be the *pdhA* gene that codes for the E1 subunit of pyruvate dehydrogenase of the *Brevibacterium lactofermentum* ATCC13869. This open reading frame, the promoter region, and a region considered to be terminator are represented in SEQ ID NO: 9 of Sequence Listing. Further, the amino acid sequence of the product deduced from this open reading frame was shown in SEQ ID NO: 9 and 10. It has been well known that a methionine residue present at an N-terminus of protein is often irrelevant with the function of the protein, and it is eliminated by the action of peptidase after the translation, since it is derived form the start codon, ATG. Also in the case of the aforementioned protein, a methionine residue at the N-terminus may have been eliminated. However, a sequence of GTG is present 6 bases upstream from ATG shown in SEQ ID NO: 7, and the amino acid may be translated from that codon.

Furthermore, pyruvate dehydrogenases of other microorganisms such as *Escherichia coli* consist of three subunits, E1, E2, and E3, and genes coding for them often constitute an operon. However, any open reading frames considered to be E2 and E3 subunits of pyruvate dehydrogenase did not exist within a region of about 3 kb downstream from the *pdhA* gene elucidated above. Instead, since it was found that a sequence estimated to be a terminator existed in the downstream of this open reading frame, it was thought that E2 and E3 subunits of the pyruvate dehydrogenase of the *Brevibacterium lactofermentum* strain ATCC13869 existed in other region on the chromosome.

### <2> Evaluation of effect for improving yield of glutamic acid production in pdhA amplified strain

### (1) Construction of plasmid for pdhA amplification

Primers shown as SEQ ID NOS: 11 and 12 were synthesized based on the nucleotide sequence of the aforementioned *pdhA* gene. PCR was performed by using these primers and chromosome of the *Brevibacterium lactofermentum* ATCC13869 prepared by using Bacterial Genomic DNA Purification Kit produced by Advanced Genetic Technologies Corp. as a template under the standard reaction conditions described in "PCR Technology", page 8, Ed. by Henry Ehrlich, Stockton Press, 1989 to amplify the *pdhA* gene. Among the synthesized primers, SEQ ID NO: 11 corresponds to a sequence of from the 1397th to 1416th nucleotides of the nucleotide sequence of the *pdhA* gene shown in SEQ ID NO: 9 of Sequence Listing. SEQ ID NO: 12 corresponds to a strand complimentary to the sequence of from 5355th to the 5374th nucleotides of SEQ ID NO: 9 in Sequence Listing, and indicated from its 5' end.

The amplified PCR product was purified in a conventional manner, and digested with restriction enzymes *Sal*I and *Eco*T22I.

On the other hand, a plasmid vector autonomously replicable in both of *Escherichia coli* cells and coryneform bacterium cells was constructed.

First, a vector having a drug resistance gene of *Streptococcus faecalis* was constructed. The kanamycin resistance gene of *Streptococcus faecalis* was amplified by PCR from a known plasmid containing that gene. The nucleotide sequence of the kanamycin resistance gene of the *Streptococcus faecalis* has already been elucidated (Trieu-Cuot, P. and Courvalin, P., Gene, 23 (3), pp.331-341 (1983)). Based on this sequence, the primers shown as SEQ ID NOS: 13 and 14 were synthesized, and PCR was performed by using pDG783 (Anne-Marie Guerout-Fleury et al., Gene, 167, pp.335-337 (1995)) as a template to amplify a DNA fragment containing the kanamycin resistance gene and its promoter.

The above DNA fragment was purified by using SUPREC02 produced by Takara Shuzo Co., Ltd., completely digested with restriction enzymes *Hind*III and *Hin*cII, and blunt-ended. The blunt-ending was performed by using Blunting Kit produced by Takara Shuzo Co., Ltd. This DNA fragment and an amplification, product obtained by PCR utilizing the primers shown as SEQ ID NOS: 15 and 16 and pHSG399 (see S. Takeshita et al., Gene, 61, pp.63-74 (1987)) as a template and purification and blunt-ending of the PCR product were mixed and ligated. The ligation reaction was performed by using DNA Ligation Kit ver.2 produced by Takara Shuzo Co., Ltd. Competent cells of *Escherichia coli* JM109 (produced by Takara Shuzo Co., Ltd.) were transformed with the ligated DNA, and applied on L medium (10 g/L of Bacto trypton, 5 g/L of Bacto yeast extract, 5 g/L of NaCl, and 15 g/L of agar, pH 7.2) containing 10 µg/ml of IPTG (isopropyl-β-D-thiogalactopyranoside), 40 µg/ml of X-Gal (5-bromo-4-chloro-3-indolyl-β-D-galactoside) and 25 µg/ml of kanamycin, and cultured overnight. Then, the formed blue colonies were picked up, and subjected to single colony isolation to obtain transformants.

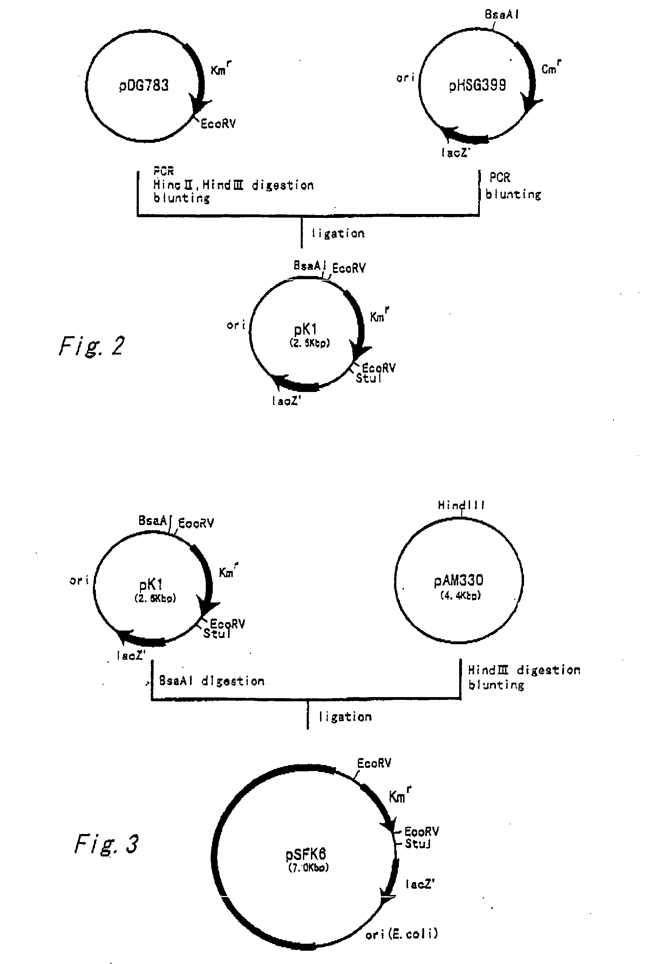

Plasmids were prepared from the transformants by using the alkaline method (Text for Bioengineering Experiments, Edited by the Society for Bioscience and Bioengineering, Japan, p.105, Baifukan, 1992), and restriction maps were prepared. A plasmid having a restriction map equivalent to the restriction map shown in Fig. 2 was designated as pK1. This plasmid is stably harbored in *Escherichia coli*, and imparts kanamycin resistance to a host. Moreover, since it contains the *lacZ'* gene, it is suitable for use as a cloning vector.

The plasmid pAM330 extracted from *Brevibacterium lactofermentum* ATCC13869 (see Japanese Patent Laid-open No. 58-67699) was completely digested with a restriction enzyme *Hin*dIII, and blunt-ended. This was ligated to the aforementioned pK1 completely digested with a restriction enzyme *Bsa*AI. *Brevibacterium lactofermentum* ATCC13869 was transformed with the ligated DNA. The transformation was performed by the electric pulse method (see Japanese Patent Laid-open No. 2-207791). Selection of transformants was performed on an M-CM2B plate (10 g/L of polypeptone, 10 g/L of yeast extract, 5 g/L of NaCl, 10 µg/L of biotin and 15 g/L of agar, pH 7.2) containing 25 µg/mL of kanamycin. After culture of 2 days, colonies were picked up, and subjected to single colony isolation to obtain transformants. Plasmids were prepared from the transformants, and restriction maps were prepared. A plasmid having the same restriction map as the restriction map shown in Fig. 3 was designated as pSFK6. This plasmid can autonomously replicate in both of *Escherichia coli* cells and coryneform bacterium cells, and imparts kanamycin resistance to a host.

The aforementioned plasmid pSFK6 was digested with restriction enzymes *Sal*I and *Pst*I. This digestion product was ligated with a *Sal*I-*Eco*T22I fragment of the aforementioned PCR product by using a ligation kit (produced by Takara Shuzo Co., Ltd.), and used for transformation of competent cells of *Escherichia coli* JM109 (produced by Takara Shuzo Co., Ltd.). The cells were applied on L medium (10 g/L of Bacto trypton, 5 g/L of Bacto yeast extract, 5 g/L of NaCl, and 15 g/L of agar, pH 7.2) containing 10 µg/ml of IPTG (isopropyl-β-D-thiogalactopyranoside), 40 µg/ml of X-Gal (5-bromo-4-chloro-3-indolyl-β-D-galactoside) and 25 µg/ml of kanamycin, and cultured overnight. Then, the formed white colonies were picked up, and subjected to single colony isolation to obtain transformants.

Plasmids were prepared from the transformants by using the alkaline method (Text for Bioengineering Experiments, Edited by the Society for Bioscience and Bioengineering, Japan, p.105, Baifukan, 1992), and restriction maps of DNA fragments inserted into the vectors were prepared and compared with the restriction map of the *pdhA* gene shown in SEQ ID NO: 9. A plasmid comprising an inserted DNA fragment having the same restriction map was designated as pSFKBPDHA.

### (2) Introduction of pSFKBPDHA into Brevibacterium lactofermentum ATCC13869 and Brevibacterium lactofermentum AJ13029 and evaluation of culture in flask

The *Brevibacterium lactofermentum* AJ13869 and the *Brevibacterium lactofermentum* AJ13029 was transformed with the plasmid pSFKBPDHA by the electric pulse method (see Japanese Patent Laid-open No. 2-207791) to obtain transformants. The obtained transformants ATCC13869/pSFKBPDHA and AJ13029/pSFKBPDHA were cultured for L-glutamic acid production as follows. Cells of AJ13029/pSFKBPDHA obtained by culture on a CM2B plate medium containing 25 µg/mL of kanamycin were inoculated to a medium containing 30 g of glucose, 1 g of KH₂PO₄, 0.4 g of MgsO₄·7H₂O, 30 g of (NH₄)₂SO₄, 0.01 g of FeSO₄· 7H₂O, 0.01 g of MnSO₄·7H₂O, 15 ml of soybean hydrolysate, 200 µg of thiamin hydrochloride, 60 µg of biotin, 25 mg of kanamycin, and 50 g of CaCO₃ in 1 L of pure water (adjusted to pH 8.0 with KOH), and cultured at 31.5°C with shaking until sugars in the medium was consumed.

The obtained culture was inoculated to a medium having the same composition as mentioned above except that biotin was not added in an amount of 5%. Then ATCC13869 was cultured at 31.5°C and AJ13029 was cultured at 37°C with shaking until sugars in the medium was consumed. As controls, the *Brevibacterium lactofermentum* strains ATCC13869 and AJ13029 transformed with pSFK6 by the electric pulse method were cultured in the same manner as described above. After the culture was completed, the amount of L-glutamic acid accumulated in the medium was measured by Biotech Analyzer AS-210 produced by Asahi Chemical Industry Co., Ltd. The results are shown in Table 4.

**Table 4**

| Strain | Yield of L-glutamic acid (%) |
|---|---|
| ATCC13869 / pSFK6 | 48.3 |
| ATCC13869 / pSFKBPDHA | 53.2 |
| AJ13029 / pSFK6 | 52.3 |
| AJ13029 / pSFKBPDHA | 59.2 |

From these results, it was found that even only the amplification of the *pdhA* gene was also effective for improving yield of L-glutamic acid production in both of the *Brevibacterium lactofermentum* ATCC13869 and the *Brevibacterium lactofermentum* AJ13029.

## Claims

1. A method for producing L-glutamic acid comprising the steps of culturing a coryneform bacterium in a medium to produce and accumulate L-glutamic acid in the medium, and collecting the L-glutamic acid from the medium, wherein said coryneform bacterium has L-glutamic acid-producing ability and has enhanced intracellular pyruvate dehydrogenase activity, wherein the intracellular pyruvate dehydrogenase activity is enhanced by increasing copy number of a gene coding for pyruvate dehydrogenase, or by replacing a promoter of the pyruvate dehydrogenase gene with a stronger promoter.

2. The method according to claim 1, wherein the gene coding for pyruvate dehydrogenase is derived from *Escherichia coli* or coryneform bacterium.

3. The method according to claim 2, wherein the pyruvate dehydrogenase is a protein defined in the following (A) or (B):
(A) a protein which has the amino acid sequence of SEQ ID NO: 10;
(B) a protein which has the amino acid sequence of SEQ ID NO: 10 including substitution, deletion, insertion, addition, or inversion of one to 20 amino acids, and has a pyruvate dehydrogenase activity.

4. The method according to claim 2, wherein the pyruvate dehydrogenase is a protein encoded by a DNA which comprises the nucleotide sequence of nucleotide numbers 2360 to 5120 in SEQ ID NO: 9.

5. The method according to any one of claims 1 to 4, wherein the medium contains vitamin B1 at 20 µg/L or more.

## Patentansprüche

1. Verfahren zur Herstellung von L-Glutaminsäure, umfassend die Schritte: Kultivieren eines coryneformen Bakteriums in einem Medium, um L-Glutaminsäure herzustellen und zu akkumulieren, und Sammeln der L-Glutaminsäure aus dem Medium, wobei das coryneforme Bakterium zur Herstellung von L-Glutaminsäure befähigt ist und erhöhte intrazelluläre Pyruvatdehydrogenaseaktivität besitzt, wobei die intrazelluläre Pyruvatdehydrogenaseaktivität durch Erhöhen der Kopienzahl eines Gens, das für Pyruvatdehydrogenase codiert, oder durch Ersetzen eines Promotors des Pyruvatdehydrogenasegens durch einen stärkeren Promotor gesteigert ist.

2. Verfahren nach Anspruch 1, wobei das Gen, das für die Pyruvatdehydrogenase codiert, von Escherichia coli oder einem coryneformen Bakterium abgeleitet ist.

3. Verfahren nach Anspruch 2, wobei die Pyruvatdehydrogenase ein Protein ist, das nachstehend in (A) oder (B) definiert ist:
(A) ein Protein, das die Aminosäuresequenz von SEQ ID NO: 10 hat;
(B) ein Protein, das die Aminosäuresequenz von SEQ ID NO: 10 hat, einschliesslich Substitution, Deletion, Insertion, Zufügung oder Inversion von 1 bis 20 Aminosäuren, und das Pyruvatdehydrogenaseaktivität besitzt.

4. Verfahren nach Anspruch 2, wobei die Pyruvatdehydrogenase ein Protein ist, das durch eine DNA codiert wird, die die Nukleotidsequenz der Nukleotidnummern 2360 bis 5120 von SEQ ID NO: 9 umfasst.

5. Verfahren nach einem der Ansprüche 1 bis 4, wobei das Medium 20 µg/ℓ oder mehr Vitamin B1 enthält.

## Revendications

1. Procédé de production d'acide L-glutamique, comprenant les étapes de culture d'une bactérie corynéforme dans un milieu pour produire et accumuler de l'acide L-glutamique dans le milieu, et de recueil de l'acide L-glutamique à partir du milieu, dans lequel ladite bactérie corynéforme a la capacité de produire de l'acide L-glutamique et a une activité de pyruvate-déshydrogénase intracellulaire renforcée, l'activité de pyruvate-déshydrogénase intracellulaire étant renforcée par l'augmentation du nombre de copies d'un gène codant pour la pyruvate-déshydrogénase, ou par le remplacement d'un promoteur du gène de pyruvate-déshydrogénase par un promoteur plus fort.

2. Procédé selon la revendication 1, dans lequel le gène codant pour la pyruvate-déshydrogénase provient d'*Escherichia coli* ou d'une bactérie corynéforme.

3. Procédé selon la revendication 2, dans lequel la pyruvate-déshydrogénase est une protéine définie dans (A) ou (B) ci-dessous:
(A) une protéine qui a la séquence d'aminoacides de SEQ ID NO: 10;
(B) une protéine qui a la séquence d'aminoacides de SEQ ID NO: 10 comprenant une substitution, une délétion, une insertion, une addition ou une inversion de 1 à 20 aminoacides, et qui a une activité de pyruvate-déshydrogénase.

4. Procédé selon la revendication 2, dans lequel la pyruvate-déshydrogénase est une protéine codée par un ADN qui comprend la séquence de nucléotides allant des numéros de nucléotides 2360 à 5120 dans SEQ ID NO: 9.

5. Procédé selon l'une quelconque des revendications 1 à 4, dans lequel le milieu contient de la vitamine B1 à raison d'au moins 20 µg/L.
